Europäisches Patentamt

European Patent Office

Office européen des brevets

(11) Publication number: **0 061 071**
**B1**

## (12) EUROPEAN PATENT SPECIFICATION

(45) Date of publication of patent specification: 29.05.85

(51) Int. Cl.⁴: **G 01 N 33/542, C 12 Q 1/26**

(21) Application number: **82101923.9**

(22) Date of filing: **11.03.82**

(54) Activated apoglucose oxidase, method of preparing it, its use in specific binding assay methods and reagent means and test kits containing it.

(30) Priority: **23.03.81 US 246591**

(43) Date of publication of application:
**29.09.82 Bulletin 82/39**

(45) Publication of the grant of the patent:
**29.05.85 Bulletin 85/22**

(84) Designated Contracting States:
**CH DE FR GB IT LI NL SE**

(56) References cited:
**EP-A-0 019 857**
**EP-A-0 027 631**
**GB-A-2 040 943**
**US-A-4 230 797**
**US-A-4 238 565**
**US-A-4 255 566**

(73) Proprietor: **MILES LABORATORIES, INC.**
**1127 Myrtle Street**
**Elkhart Indiana 46514 (US)**

(72) Inventor: **Morris, David Lindsay**
**23274 Ronda Drive**
**Elkhart, IN 46514 (US)**

(74) Representative: **Senftl, Hannes, Dr. et al**
**c/o Bayer AG Konzernverwaltung RP**
**Patentabteilung**
**D-5090 Leverkusen Bayerwerk (DE)**

Courier Press, Leamington Spa, England.

## Description

### Background of the invention
### 1. Field of the invention

This invention relates to a method for increasing the ability of apoglucose oxidase to combine with flavin adenine dinucleotide (FAD) and FAD derivatives to form active glucose oxidase, i.e., a method for activating apoglucose oxidase. The invention further relates to homogeneous specific binding assay methods, reagent means, and test kits for determining a ligand, such as an antigen, hapten or antibody, in a liquid medium wherein FAD is employed as label and wherein the FAD label is monitored by its ability to combine with apoglucose oxidase to form active glucose oxidase.

Glucose oxidase is a conjugated enzyme composed of an enzymatically inactive, high molecular weight protein component (apoenzyme) and FAD (a low molecular weight, nonproteinaceous prosthetic group). Apoglucose oxidase and FAD have a high binding affinity (binding constant of around $10^{10}$ molar$^{-1}$), but can be effectively separated by treatment with acidified ammonium sulfate [Swoboda, *Biochim. Biophys. Acta 175*: 365—379 (1969)]. Swoboda demonstrated that apoglucose oxidase exists in solution mainly with the molar configuration of a loose flexible coil, and to a lesser extent in a compact globular form, whereas when FAD is added the protein is converted to a compact, nearly spherical form having glucose oxidase activity. FAD is understood to most efficiently bind with the globular form of apoglucose oxidase to yield active glucose oxidase. Stabilization of apoglucose oxidase by intramolecular crosslinking with glutaraldehyde has been reported [Solomon *et al, Biopolymers 16*: 1837—1952 (1977)].

### 2. Brief description of the prior art

U.S. Patent No. 4,238,565 assigned to the present assignee describes specific binding assay methods wherein FAD is employed as label and is monitored by its ability to combine with apoglucose oxidase to form active glucose oxidase. Both homogeneous and heterogeneous formats are anticipated. In a homogeneous assay for determining an antigen in a liquid medium, a test sample of the liquid medium is combined with antibody to the antigen and with a labeled conjugate comprising the antigen or an analog thereof coupled to FAD whereby any antigen from the sample competes with antigen-FAD for binding with antibody. Apoglucose oxidase is also present or is added after an appropriate incubation period and is capable of combining with antigen-FAD which has not been bound by antibody to yield active glucose oxidase. However, antibody-bound antigen-FAD is not capable of such combination with apoglucose oxidase. Consequently, the concentration of antigen in the test sample dictates the amount of measurable glucose oxidase which results.

Glucose oxidase activity is measurable in a wide variety of known manners, including colorimetric methods.

The FAD-labeled homogeneous specific binding assay is generally applicable to the determination of a wide variety of ligands over a wide range of concentrations. However, application of the assay method to certain existing analytical instrumentation requiring incubation of the assay reaction mixture at elevated temperatures and for short periods has been found to be limited. It has been found that the ability of FAD and FAD-derivatives (i.e., ligand-FAD conjugates) to combine with apoglucose oxidase to form active glucose oxidase decreases rapidly with increasing temperature above room temperature, to the point that at temperatures above about 30°C significantly greater amounts of apoglucose oxidase must be added to the assay reaction mixture to obtain a dose-response curve. At 35—40°C, the FAD-apoglucose oxidase recombination reaction is slowed to the point that analytically useful dose-response curves are not possible to obtain with short incubation periods (e.g., 10 minutes or less) for the determination of ligands at concentrations below $10^{-6}$M.

The preparation of antibody to glucose oxidase is reported by Green *et al, J. Immunol. 104*: 1094—1100 (1970). Apoglucose oxidase preparations useful in the FAD-labeled specific binding assay method are described in U.S. Patent Application Serial No. 45,191, filed June 4, 1979 and assigned to the present assignee.

### Summary of the invention

It has now been found that the ability of apoglucose oxidase to combine with FAD to form active glucose oxidase is increased by interacting apoglucose oxidase with a substance (hereinafter referred to as "anti-glucose oxidase") which is an antibody raised against glucose oxidase or a fragment thereof having a specific binding affinity for glucose oxidase, e.g., Fab, F(ab'), or F(ab')$_2$.

The apoglucose oxidase/anti-glucose oxidase complex has been found to be activated to recombination with FAD. The apoglucose oxidase activation has been found to increase with increasing temperatures between 25°—37°C to the extent that at 37°C the formation of active glucose oxidase is enhanced as much as 100 to 1000-fold by the presence of antiglucose oxidase. Thus, FAD-labeled homogeneous specific binding assays for ligands appearing in concentrations between $10^{-5}$ and $10^{-8}$M are made feasible for use with analytical instrumentation requiring elevated reaction temperatures (e.g., 37°C) and short incubation periods (e.g., 5—7 minutes).

### Brief description of the drawings

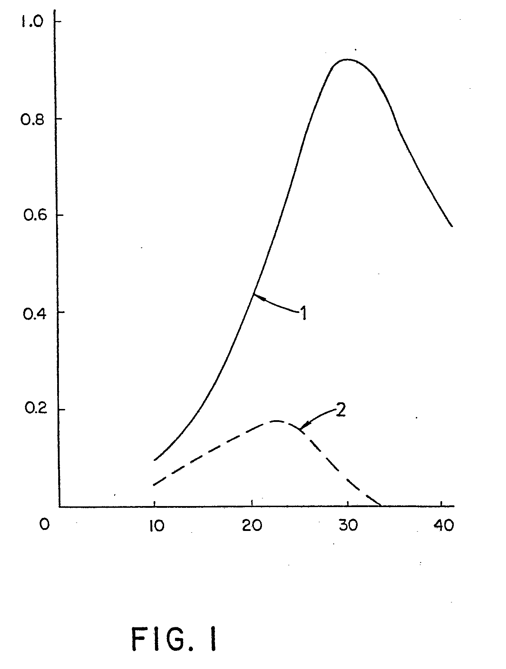

Fig. 1 is a graphical representation of data from the examples set forth below showing the effect of temperature on the FAD/apoglucose oxidase recombination reaction in the presence and absence of anti-glucose oxidase. The absorbance at 520 nm is plotted against the temperature (°C). Solid line (1) represents anti-glucose oxidase serum; broken line (2) represents nonimmune serum.

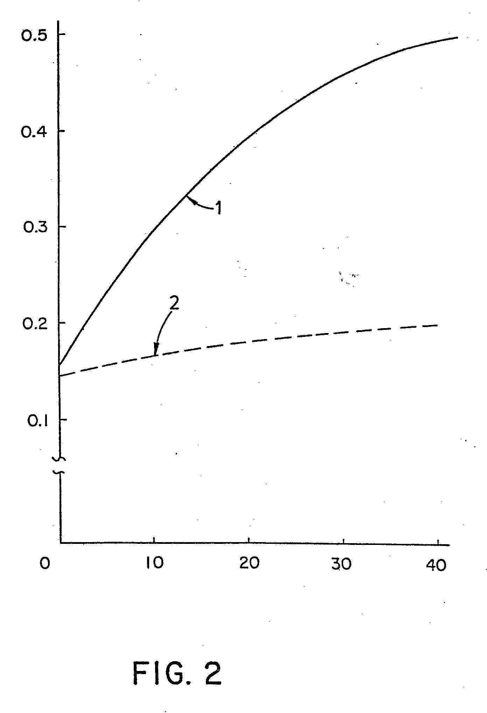

Fig. 2 is a graphical representation of data from the examples below showing optimized standard curves for a theophylline assay at 37°C with and without anti-glucose oxidase. The absorbance at 520 nm is plotted against the theophylline concentration (µg/ml). Curve (1) represents the assay with and curve (2) without antiglucose oxidase.

Description of the preferred embodiments

In the context of this disclosure, the following terms shall be defined as follows unless otherwise indicated:

Ligand—the analyte which is the subject of an assay, i.e., the substance, or class of related substances, whose presence or amount in a liquid medium is under determination.

Specific binding partner of the ligand—any substance, or class of substances, which has a specific binding affinity for the ligand to the exclusion of other substances.

FAD-labeled specific binding assay—an assay, usually an immunoassay, of any of the homogeneous types described in the aforesaid U.S. Patent No. 4,238,565 incorporated herein by reference wherein the label is FAD.

Reagent means—a composition, test device or test kit comprising the reagents used to perform an FAD-labeled specific binding assay.

The anti-glucose oxidase of the present invention may be an antibody or an antibody fragment having a specific binding affinity for glucose oxidase. When in the form of whole antibody, anti-glucose oxidase may be of any of the known classes, e.g., IgG, IgM, and so forth, and of any subclasses thereof. Any fragment of any such antibody which retains the specific binding affinity for glucose oxidase may also be employed, for instance, the fragments of IgG conventionally known as Fab, F(ab'), and F(ab')$_2$. Antibodies to glucose oxidase may be obtained by any known means. In particular, such antibodies may be obtained in the form of antiserum from an animal (e.g., mouse, rabbit, guinea pig, goat, etc.) which has been immunized against glucose oxidase. Such antibodies can also be obtained by somatic cell hybridization techniques, such antibodies being commonly referred to as monoclonal antibodies. Reviews of such monoclonal antibody techniques are found in *Lymphocyte Hybridomas*, ed. Melchers *et al*, Springer-Verlag (New York 1978), *Nature 266*: 495 (1977), and *Science 208*: 692 (1980). Activation of apoglucose oxidase by anti-glucose oxidase is believed to result from the formation of an immune complex which tends to hold the apoenzyme in a molecular configuration favoring the formation of active glucose oxidase upon binding with FAD.

The amount of anti-glucose oxidase to be added to a given amount of apoglucose oxidase to give optimum activation will be determined empirically. It has been found in general that as increasing amounts of anti-glucose oxidase are added to a fixed amount of apoglucose oxidase, apoenzyme activation increases until a maximum activation is attained. The further addition of anti-glucose oxidase does not result in further activation, but rather in general actually leads to decreased activation. Excess anti-glucose oxidase accordingly will not be desirable. Titration of anti-glucose oxidase against glucose oxidase activity will indicate for a given amount or concentration of apoenzyme the optimum amount or concentration of anti-glucose oxidase to use.

The ability of anti-glucose oxidase to activate apoglucose oxidase occurs over a reasonably broad temperature range, running between 0°C and temperatures at which the proteins denature significantly. The activation is particularly significant over the range of 10°C to 45°C, most particularly between about 30·C and about 40°C.

The ability to activate apoglucose oxidase with anti-glucose oxidase provides particular advantages in application to FAD-labeled homogeneous specific binding assays. Such assays are basically characterized by the monitoring of the FAD label by its ability to combine with apoglucose oxidase to form active glucose oxidase. In accordance with the present invention, such apoglucose oxidase is activated by interaction with anti-glucose oxidase. The present invention is particularly applicable to an FAD-labeled homogeneous immunoassay method for determining a ligand in a liquid medium wherein a reaction mixture is formed by combining the liquid medium with reagent means including (a) a labeled conjugate comprising the FAD label coupled to said ligand or a binding analog thereof, i.e., a substance which is bound by the ligand binding partner substantially the same as the ligand, (b) an antibody to the ligand, and (c) apoglucose oxidase, and wherein glucose oxidase activity is thereafter measured in the reaction mixture as a direct function of the amount of ligand in the liquid medium under assay. Such assay method can be used to determine such ligands as antigenic proteins, including antibodies, and polypeptides having molecular weights between 1,000 and 10,000,000 and haptens having molecular weights between 100 and 1,500.

Alternatively, where the ligand under determination can bind with an FAD-labeled binding partner to the ligand to affect the ability of the FAD label to combine with apoglucose oxidase, such as where the ligand is a binding protein and the labeled binding partner is the substance bound thereby, the present invention is applicable to an FAD-labeled homogeneous immunoassay method for determining such as a ligand in a liquid medium wherein a reaction mixture is formed by combining the liquid medium with reagent means including (a) a labeled conjugate comprising the FAD label coupled to said binding partner of the ligand and (b) apoglucose oxidase, and wherein glucose oxidase activity thereafter measured in the reaction mixture is an inverse function of the amount of ligand in the liquid medium under assay. Such ligand/binding partner pairs to which such a method is applicable include antibodies/hapten or antigen, thyroxine binding globulin/thyroxine or triiodothyronine, and the like.

When used in an FAD-labeled specific binding assay, apoglucose oxidase activation may be obtained by a variety of ways of interacting the apoenzyme with anti-glucose oxidase. In one embodiment, anti-glucose oxidase and the principal reagents of the assay, usually comprising (a) an FAD-labeled form of the ligand or an analog thereof, (b) a specific binding partner of the ligand, e.g., an antibody, (c) apoglucose oxidase, and (d) a glucose oxidase indicator system, are combined as separate reagents to form the assay reaction mixture. In another embodiment, some of the reagents are pre-combined, e.g., (a) apoglucose oxidase and the binding partner and (b) FAD-labeled conjugate and the glucose oxidase indicator system, and added to the others as combined reagents.

The anti-glucose oxidase and apoglucose oxidase may be added to the assay reaction mixture in the form of their activated complex. In cases of short assay incubation times and low analyte concentrations this may be preferable since in such a case essentially all of the apoglucose oxidase is in its activated form immediately upon formation of the assay reaction mixture. If added as separate reagents, the apoenzyme and anti-glucose oxidase require some finite time for complete activation of apoglucose oxidase by complexing with the anti-glucose oxidase. Where an apoglucose oxidase/anti-glucose oxidase complex reagent is used, it may be preferable to employ monovalent antibody fragments, e.g., Fab or F(ab'), as the anti-glucose oxidase. In this way one can prevent the possible formation of intermolecular crosslinks between apoglucose oxidase which otherwise could lead to the formation of suspensions or precipitates which could interfere with the assay reaction or the measured signal.

The reagent means of the present invention comprises all of the essential chemical elements required to conduct a desired assay method of the present invention. Such reagent means are presented in a commercially packaged form, as a composition or admixture where the compatability of the reagents will allow, in a test device configuration of any present or future type, or as a test kit, i.e., a packaged combination of one or more containers or vessels holding the necessary reagents. The reagents of the present invention include (a) an FAD-labeled conjugate comprising FAD coupled to the ligand, a binding analog thereof, or a specific binding partner thereof, depending on the particular assay technique intended, (b) apoglucose oxidase, (c) where the FAD-labeled conjugate comprises the ligand or an analog thereof, a specific binding partner of the ligand (e.g., an antibody or other binding protein), and (d) anti-glucose oxidase as defined herein, and optionally one or more or all of an indicator composition for detecting glucose oxidase activity (e.g., comprising glucose, peroxidase and a chromogen or chromogen system responsive to hydrogen peroxide produced when glucose oxidase acts on glucose). Of course, the reagent means can include other substances as are known in the art and which may be desirable from a commercial and user standpoint, such as buffers, diluents, standards, and so forth.

The present invention will now be illustrated, but is not intended to be limited by the following examples.

Apoglucose oxidase substantially free from residual glucose oxidase activity is prepared by the method described in U.S. Patent Application Serial No. 45,191, filed June 4, 1979 and assigned to the present assignee.

Theophylline-FAD conjugates are prepared by the method described in U.S. Patent No. 4,238,565 assigned to the present assignee.

Sodium 3,5 - dichloro - 2 - hydroxybenzene sulfonate (sodium DHSA) is prepared by hydrolysis of sodium 3,5 - dichloro - 2 - hydroxybenzene sulfonyl chloride (Aldrich Chemical Co., Milwaukee, WI) with sodium bicarbonate solution.

Antibody to glucose oxidase is obtained by injecting goats on four subcutaneous sites near the shoulders with a total of 3.0 ml adjuvant (1.5 ml Freund's adjuvant and 1.5 ml saline) containing glucose oxidase in the range 0.1—1.0 mg. Complete Freund's adjuvant is used for the initial injection and incomplete Freund's adjuvant is used for the booster injections. Five weeks after the initial injection, the following schedule is followed at one week intervals: boost, bleed, bleed, rest, repeat.

Effect of temperature on the activation of apoglucose oxidase in the presence and absence of antiserum against glucose oxidase

A. Reagents

Mix A

2 micromolar (μM) apoglucose oxidase in 0.1 molar (M) sodium phosphate buffer, pH 7.0, containing 4 millimolar (mM) aminophenazone, 10% glycerol, and 0.1% (w/v) bovine serum albumin. This mix was incubated at room temperature.

Mix B

1.0 nanomolar (nM) theophylline-FAD was added to a glucose oxidase assay reagent solution consisting of 0.1 M sodium phosphate buffer, pH 7.0, 0.105 M glucose, 2.11 mM sodium DHSA, 1.05% (w/v) bovine serum albumin and 63 micrograms per milliliter (μg/ml) peroxidase. Aliquots of this mix were incubated at the various temperatures indicated below.

B. Assays

Mix A [100 microliters (µl)] was mixed with either 10 µl of goat antiserum against glucose oxidase (anti-GO serum) or 10 µl of nonimmune goat serum (NI serum) in disposable cuvettes. The solutions were incubated at room temperature for 15 minutes. Mix B (1.90 ml) equilibrated to the desired assay temperature was then added and the reaction mixture mixed by inversion. After a 15 minute incubation at the desired assay temperature, the absorbance at 520 nanometers (nm) was read against Mix B. Blanks were also run exactly as above except that theophylline-FAD was absent from Mix B. The results are shown in tabular form below and as a graph in Figure 1 of the drawings. The absorbance readings are corrected for the blank.

| Temperature (°C) | Absorbance (520 nm) | |
|---|---|---|
| | NI serum | Anti-GO serum |
| 10 | 0.041 | 0.094 |
| 15 | 0.100 | 0.212 |
| 20 | 0.134 | 0.430 |
| 24 | 0.170 | 0.682 |
| 28 | 0.073 | 0.885 |
| 32 | 0.020 | 0.929 |
| 36 | NC* | 0.778 |
| 40 | NC* | 0.606 |

*no measurable color

These data demonstrate that the presence of anti-glucose oxidase antiserum enhances the ability of apoglucose oxidase to combine with the theophylline-FAD conjugate to form active glucose oxidase. At room temperature (25°C), the presence of anti-glucose oxidase produces a 4-fold increase in apoglucose oxidase activation. The effect at 37°C is estimated at between 100 and 1000-fold.

Theophylline immunoassay at 37°C in the presence and absence of antiserum to glucose oxidase
I. Assay in the absence of anti-glucose oxidase

Reagents:
Mix A—Combined apoenzyme/anti-theophylline reagent: 32 µM apoglucose oxidase and 50 µl anti-theophylline antiserum per ml were added to 0.1 M sodium phosphate buffer, pH 7.0, containing 0.1% (w/v) bovine serum albumin. This mix was incubated at 20°C.

Mix B—combined glucose oxidase assay and theophylline-FAD label: 63 nM theophylline-FAD was added to 0.1 M sodium phosphate buffer, pH 7.0, containing 0.105 M glucose, 2.11 mM sodium DHSA, 0.21 mM aminophenazone, 1.05% (w/v) bovine serum albumin, and 63 µg/ml horseradish peroxidase. This mix was incubated at 37°C.

Theophylline standards: Prepared by gravimetrically spiking pooled human serum with different amounts of theophylline. These standards were diluted 10-fold with 0.1 M sodium phosphate buffer, pH 7.0.

Assay procedure: Mix B (1.90 ml) was mixed with 50 µl of a diluted theophylline standard in a disposable cuvette and equilibrated at 37°C. Mix A (0.10 ml) was then added and the reaction mixture mixed by inversion. After a 6 minute incubation at 37°C, the absorbance at 520 nm was read against Mix B. The final assay concentrations for the principal reagents were:

| | |
|---|---|
| apoglucose oxidase | 1.60 µM |
| theophylline-FAD | 60 nM |
| anti-theophylline | 2.5 µl/ml |

The results are shown in tabular form below and as a graph in Figure 2 of the drawings.

| Theophylline serum concentration (µg/ml) | Absorbance (520 nm) |
|---|---|
| 0 | 0.146 |
| 6 | 0.162 |
| 12 | 0.174 |
| 20 | 0.183 |
| 30 | 0.190 |
| 40 | 0.198 |

The absorbance change over the full range of theophylline serum concentrations was only 0.052. This data represents the present optimized theophylline assay in the absence of anti-glucose oxidase.

II. Assay in the presence of anti-glucose oxidase

Reagents:
Mix A—600 nM apoglucose oxidase, 100 µl/ml rabbit antiserum against glucose oxidase, and 20 µl/ml anti-theophylline were added to 0.1 M phosphate buffer, pH 7.0, containing 4 mM aminophenazone and 10% glycerol. This mix was incubated at room temperature.

Mix B—10.5 nM theophylline-FAD was added to 0.10 M phosphate buffer, pH 7.0, 0.105 M glucose, 2.11 mM sodium DHSA, 1.05% (w/v) bovine serum albumin, and 63 µg/ml horseradish peroxidase. This mix was incubated at 37°C.

Theophylline standards: Same as above.

Assay procedure: Mix B (1.90 ml) was mixed with 20 µl of a diluted theophylline standard in a disposable cuvette and equilibrated at 37°C. Mix A (0.10 ml) was then added and the reaction mixture mixed by inversion. After a 5 minute incubation at 37°C, the absorbance at 520 nm was read against Mix B. The final assay concentrations for the principal reagents were:

| | |
|---|---|
| apoglucose oxidase | 0.03 µM |
| theophylline-FAD | 10 nM |
| anti-theophylline | 1.0 µl/ml |

The results are shown in tabular form below and as a graph in Figure 2 of the drawings.

| Theophylline serum concentration (µg/ml) | Absorbance (520 nm) |
|---|---|
| 0 | 0.162 |
| 10 | 0.298 |
| 20 | 0.378 |
| 30 | 0.444 |
| 40 | 0.495 |

The absorbance change over the full range of theophylline serum concentrations was 0.333 compared to 0.052 for the optimized assay without anti-glucose oxidase (part I above). Further, the optimized theophylline assay at 37°C with anti-glucose oxidase present required approximately 50-fold less apoglucose oxidase, 6-fold less theophylline-FAD conjugate, and 2.5-fold less anti-theophylline. These data demonstrate the greatly improved features of the optimized assay at 37°C with anti-glucose oxidase present over that without anti-glucose oxidase.

Use of antibody fragments to activate apoglucose oxidase
I. Preparation of anti-glucose oxidase IgG

Goat antiserum against glucose oxidase (35 ml) was dialysed against 2 liters of 15 mM potassium phosphate, pH 8.0, for 16 hours at room temperature. The dialysed solution was applied to a column (2.5×30 cm) DE-52 cellulose (Whatman Ltd., UK) equilibrated with 15 mM potassium phosphate, pH 8.0. The column was eluted with the same buffer at a flow rate of 50 ml/hour and 12 ml fractions were collected. The absorbance of the effluent was monitored at 280 nm. An initial absorbance peak was eluted in fractions 14 to 21 and this was pooled and labeled "FI". The eluting buffer was changed to 50 mM potassium phosphate, pH 8.0, and a second major absorbance peak was collected in fractions 39 to 44 which was pooled and labeled "FII". The two absorbance peaks correlated well with the ability of the fractions to activate apoglucose oxidase.

II. Papain digestion of anti-glucose-oxidase IgG (Fab fragments)

Protein from FI (50 mg) was incorporated into 5 ml of 50 mM sodium phosphate, pH 7.3, containing 0.15 M sodium chloride, 25 mM cysteine, 1 mM ethylenediamine tetraacetic acid (EDTA) and 1 mg papain. The solution was incubated at 37°C for 60 minutes. Solid iodoacetamide (28 mg) was then added to give a 30 mM solution for the purpose of inactivating the papain. This solution was incubated for a further 15 minutes after which it was applied to a column (25×90 cm) of Sephadex G150 equilibrated with 50 mM sodium phosphate, pH 7.0, containing 0.02% (w/v) sodium azide. The column was developed by elution with the same buffer at a flow rate of 25 ml/hour and 4.6 ml fractions were collected. Absorbance at 280 nm was monitored and a peak eluted between fractions 47 to 72 was shown to contain a mixture of Fab and Fc fragments and correlated well with the ability of the fractions to activate apoglucose oxidase. This material was pooled, concentrated to 7.9 mg/ml protein and labeled "FI(a)". No whole igG was eluted and it was concluded that complete digestion of IgG had occurred.

Protein from FII (50 mg) was digested as described above. Chromatography on Sephadex G150 was performed as described above. Absorbance peaks at 280 nm were eluted, one between fractions 34 to 50 which was identified as IgG and a second between fractions 56—75 which was shown to contain a mixture of Fab and Fc fragments. Both absorbance peaks correlated well with the ability of the fractions to activate apoglucose oxidase. The first peak was pooled, concentrated to 16.8 mg/ml and labeled "FII(a)". The second peak was pooled, concentrated to 6.2 mg/ml and labeled "FII(b)".

III. Activation of apoglucose oxidase by Fab fragments

A. Reagents

Apoglucose oxidase (3.2 µM) was prepared in 0.1 M sodium phosphate buffer, pH 7.0, containing 8 mM aminophenazone and 10% glycerol. The glucose oxidase assay reagent contained 0.10 M sodium phosphate buffer, pH 7.0, 0.105 M glucose, 2.11 mM sodium DHSA, 1.05% (w/v) bovine serum albumin, 63 µg/ml peroxidase, and 1.0 nM theophylline-FAD.

B. Assays

Aliquots (0.20 ml) of 0.10 M sodium phosphate buffer, pH 7.0, containing antibody or antibody fragments were mixed with 0.03 ml of apoglucose oxidase reagent in a disposable cuvette and incubated at room temperature for 15 minutes.

The glucose oxidase reagent (1.90 ml) was equilibrated at 37°C and added to the cuvette. The assay solution was incubated at 37°C for 15 minutes and the absorbance read at 520 nm against the glucose oxidase reagent solution as reference. The recorded absorbances were corrected for a blank (assay solution without theophylline-FAD).

The results were as follows:

Activation by FI(a)—Fab and Fc fragments

| Protein (µg) | Absorbance (520 nm) |
|---|---|
| 0 | 0.050 |
| 80 | 0.300 |
| 160 | 0.555 |
| 320 | 0.700 |
| 400 | 0.900 |
| 800 | 0.922 |
| 1600 | 0.778 |

Activation by FII(a)—whole IgG

| Protein (µg) | Absorbance (520 nm) |
|---|---|
| 0 | 0.050 |
| 84 | 0.405 |
| 168 | 0.779 |
| 252 | 0.910 |
| 420 | 1.044 |
| 840 | 1.094 |
| 1680 | 1.140 |

Activation by FII(b)—Fab and Fc fragments

| Protein (µg) | Absorbance (520 nm) |
|---|---|
| 0 | 0.050 |
| 62 | 0.257 |
| 124 | 0.504 |
| 186 | 0.693 |
| 310 | 0.918 |
| 620 | 1.046 |
| 1240 | 1.000 |

These data demonstrate that fragments (Fab) of anti-glucose oxidase antibody are effective in activating apoglucose oxidase.

**Claims**

1. A complex of apoglucose oxidase and an antibody, or a fragment thereof, to glucose oxidase.

2. A method for increasing the ability of apoglucose oxidase to combine with flavin adenine dinucleotide and derivatives thereof to form active glucose oxidase comprising the step of interacting apoglucose oxidase with an antibody, or a fragment thereof, to glucose oxidase.

3. The method of Claim 2 wherein said antibody, or fragment thereof, is an antibody of the IgG class, or a fragment thereof.

4. A homogeneous specific binding assay method for determining a ligand in a liquid medium wherein flavin adenine dinucleotide is employed as label and is monitored by its ability to combine with apoglucose oxidase to form active glucose oxidase, characterized in that said apoglucose oxidase is interacted with an antibody, or a fragment thereof, to glucose oxidase.

5. The method of Claim 4 wherein said antibody, or fragment thereof, is an antibody of the IgG class, or a fragment thereof.

6. A homogeneous immunoassay method for determining a ligand in a liquid medium, wherein a reaction mixture is formed by combining said liquid medium with reagent means including

(a) a labeled conjugate comprising, as label, flavin adenine dinucleotide coupled to said ligand or a binding analog thereof,

(b) an antibody to said ligand, and

(c) apoglucose oxidase,

and wherein glucose oxidase activity is measured in said reaction mixture, characterized in that an antibody, or a fragment thereof, to glucose oxidase is added to said reaction mixture.

7. The method of Claim 6 wherein said antibody, or fragment thereof, is added to said reaction mixture in the form of its immune complex with said apoglucose oxidase.

8. A reagent means for determining a ligand in a liquid medium by a homogeneous specific binding assay method, which means includes (a) a labeled conjugate wherein the label is flavin adenine dinucleotide and (b) apoglucose oxidase, characterized in that an antibody, or a fragment thereof, to glucose oxidase is included in said means.

9. A test kit for determining a ligand in a liquid medium, comprising

(1) a labeled conjugate comprising, as label, flavin adenine dinucleotide coupled to said ligand or a binding analog thereof,

(2) a specific binding partner of said ligand,

(3) apoglucose oxidase, and

(4) an antibody, or a fragment thereof, to glucose oxidase.

10. A test kit for determining a ligand in a liquid medium, comprising

(1) a labeled conjugate comprising, as label, flavin adenine dinucleotide coupled to a specific binding partner of said ligand,

(2) apoglucose oxidase, and

(3) an antibody, or a fragment thereof, to glucose oxidase.

**Revendications**

1. Complexe d'apoglucose oxydase et d'un anticorps ou d'un fragment d'un anticorps contre la glucose oxydase.

2. Procédé pour accentuer l'aptitude de l'apoglucose oxydsae à se combiner avec le flavine adénine dinucléotide et ses dérivés pour former de la glucose oxydase active, caractérisé en ce qu'il comprend l'interaction de l'apoglucose oxydase avec un anticorps ou un fragment d'un anticorps contre la glucose oxydase.

3. Procédé suivant la revendication 2, caractérisé en ce que ledit anticorps ou son fragment est un anticorps ou un fragment d'un anticorps de la classe IgG.

4. Méthode d'analyse par liaison spécifique homogène pour le dosage d'un ligand dans un milieu liquide où le flavine adénine dinucléotide est utilisé comme marqueur et est contrôlé par son apittude à se combiner avec l'apoglucose oxydase pour former de la glucose oxydase active, caractérisée en ce que ladite apoglucose oxydase est soumise à l'interaction avec un anticorps ou un fragment d'un anticorps contre la glucose oxydase.

5. Méthode suivant la revendication 4, caractérisée en ce que ledit anticorps ou son fragment est un anticorps ou un fragment d'un anticorps de la classe IgG.

6. Méthode d'analyse immunologique homogène pour le dosage d'un ligand dans un milieu liquide, caractérisé en ce qu'un mélange réactionnel est formé par la réunion du milieu liquide avec un réactif comprenant

(a) un conjugué marqué ayant comme marqueur du flavine adénine dinucléotide couplé audit ligand ou à son analogue de liaison,

(b) un anticorps contre ledit ligand, et

(c) de l'apoglucose oxydase,

et l'activité en glucose oxydase est mesurée dans ledit mélange réactionnel, caractérisée en ce qu'un anticorps ou un fragment d'un anticorps contre la glucose oxydase est ajouté audit mélange réactionnel.

7. Méthode suivant la revendication 6, caractérisée en ce que ledit anticorps ou son fragment est ajouté audit mélange réactionnnel sous la forme de son complexe immun avec ladite apoglucose oxydase.

8. Réactif pour le dosage d'un ligand dans un milieu liquide par un méthode d'analyse par liaison spécifique homogène, qui comprend (a) un conjugué marqué dont le marqueur est le flavine adénine dinucléotide et (b) de l'apoglucose oxydase, caractérisé en ce qu'un anticorps ou un fragment d'un anticorps contre la glucose oxydase y est inclus.

9. Kit d'analyse pour le dosage d'un ligand dans un milieu liquide, caractérisé en ce qu'il comprend

(1) un conjugué marqué renfermant comme marqueur le flavine adénine dinucléotide couplé audit ligand ou à son analogue de liaison,

(2) un partenaire de liaison spécifique dudit ligand,

(3) de l'apoglucose oxydase, et

(4) un anticorps ou un fragment d'un anticorps contre la glucose oxydase.

10. Kit d'analyse pour le dosage d'un ligand dans un milieu liquide, caractérisé en ce qu'il comprend

(1) un conjugué marqué ayant comme marqueur le flavine adénine dinucléotide couplé à un partenaire de liaison spécifique dudit ligand,

(2) d l'apoglucose oxydase, et

(3) un anticorps ou un fragment d'un anticorps contre la glucose oxydase.

**Patentansprüche**

1. Apoglucose-oxidase-Komplex und Antikörper oder ein Fragment desselben für Glucose-oxidase.

2. Verfahren zur Erhöhung der Bindungsfähigkeit von Apoglucose-oxidase mit Flavin-adenin-dinucleotid und Derivaten desselben unter Bildung von aktiver Gucose-oxidase, gekennzeichnet durch eine Reaktion bzw. Wechselwirkung von Apoglucose-oxidase mit einem Antikörper oder einem Fragment desselben für Glucose-oxidase.

3. Verfahren nach Anspruch 2, dadurch gekennzeichnet, dass der genannte Antikörper oder ein Fragment desselben einen Anti-körper der IgG-Klasse oder ein Fragment desselben darstellt.

4. Homogenes spezifisches Bindungs-Assay-Verfahren zur Bestimmung eines Liganden in einem flüssigen Medium, wobei Flavin-adenin-dinucleotid also Marker verwendet und dessen Bindungsfähigkeit mit Apoglucose-oxidase unter Bildung von aktiver Glucose-oxidase registriert wird, dadurch gekennzeichnet, dass die genannte Apoglucose-oxidase in Wechselwirkung mit einem Antikörper oder einem Fragment desselben für Glucose-oxidase gebracht wird.

5. Verfahren nach Anspruch 4, dadurch gekennzeichet, dass der genannte Antikörper oder ein Fragment desselben einen Antikörper der IgG-Klasse oder ein Fragment desselben darstellt.

6. Homogenes Immuno-Assay-Verfahren zur Bestimmung eines Liganden in einem flüssigen Medium, wobei ein Reaktionsgemisch gebildet wird durch Kombination des genannten flüssigen Mediums mit Reagenzien, enthaltend

(a) ein markiertes Konjugat, welches als Marker Flavin-adenin-dinucleotid, das an den genannten Liganden oder ein Bindungsanalogon desselben gekoppelt ist, umfasst

(b) einen Antikörper für den genannten Liganden, und

(c) Apoglucose-oxidase,

und wobei die Glucose-oxidase-Aktivität in dem genannten Reaktionsgemisch gemessen wird, dadurch gekennzeichnet, dass ein Antikörper oder ein Fragment desselben für Glucose-oxidase zu dem genannten Reaktionsgemisch zugegeben wird.

7. Verfahren nach Anspruch 6, dadurch gekennzeichnet, dass der genannte Antikörper oder ein Fragment desselben zu dem genannten Reaktionsgemisch in Form eines Immun-Komplexes desselben mit der genannten Apoglucose-oxidase zugegeben wird.

8. Reagens zur Bestimmung eines Liganden in einem flüssigen Medium durch ein homogenes spezifisches Bindungs-Assay-Verfahren, welches

(a) ein markiertes Konjugat, in welchem der Marker Flavin-adenin-dinucleotid darstellt, und

(b) Apoglucose-oxidase umfasst,
dadurch gekennzeichnet, dass das genannte Reagens einen Antikörper oder ein Fragment desselben für Glucose-oxidase enthält.

9. Testkit zur Bestimmung eines Liganden in einem flüssigen Medium, welcher

(1) ein markiertes Konjugat, das als Marker Flavin-adenin-dinucleotid umfasst, welches an den genannten Liganden oder an ein Bindungsanalogon desselben gekoppelt ist,

(2) einen spezifischen Bindungspartner des genannten Liganden,

(3) Apoglucose-oxidase, und

(4) einen Antikörper oder ein Fragment desselben für Glucose-oxidase
umfasst.

FIG. I

FIG. 2